# EUROPEAN PATENT SPECIFICATION

(11) **EP 1 021 737 B1**
(45) Date of publication and mention of the grant of the patent: **11.06.2008**
(21) Application number: 98950838.7
(22) Date of filing: 02.10.1998
(51) Int. Cl.: G02B 6/02, A61B 18/22

(54) **APPARATUS FOR ABLATING TISSUE**
VORRICHTUNG ZUR GEWEBEABLATION
DISPOSITIF D'ABLATION DE TISSUS

(30) Priority: 06.10.1997 US 943961
(43) Date of publication of application: 26.07.2000
(73) Proprietor: Acculase, Inc., San Diego, CA 95035 (US)
(72) Inventor: HARTMAN, Raymond, A., Carlsbad, CA 92009 (US)
(74) Representative: Baker, Colin John
(86) International application number: PCT/US1998/020648
(87) International publication number: WO 1999/018460

(56) References cited:
- EP-A- 0 797 957
- CA-A- 2 201 670
- US-A- 4 266 548
- US-A- 4 988 163
- US-A- 5 562 658

## Description

### FIELD OF THE INVENTION

The present invention is directed to surgical methods and apparatus for ablating tissue. More particularly, the present invention is directed to surgical methods and apparatus for revascularizing tissue, for example, heart tissue, with laser energy in a consistent, precise, and programmable manner through the use of a laser tissue drill.

### BACKGROUND OF THE INVENTION

Cardiomyopathy (*cardio* meaning "heart" and *myopathy* meaning "muscle disease") refers to a group of disorders that directly damage the muscle of the heart walls, or *myocardium.* In these disorders, all chambers of the heart are affected. The heart's function as a pump is disrupted, leading to an inadequate blood flow to organs and tissues of the body. Depending on the nature of the injury or abnormality in the heart muscle and the resulting structural changes in the heart chambers, one of three types of nonischemic (that is, not caused by heart attack) heart muscle disease may be present in a patient: *dilated congestive, hypertrophic,* or *restrictive.*

Dilated congestive cardiomyopathy damages the fibers of the heart muscle, weakening the walls of the heart's chambers. The chambers thereby lose some of their capacity to contract forcefully and pump blood through the circulatory system. To compensate for the muscle injury, the heart chambers enlarge or dilate which causes heart failure. Hypertrophic cardiomyopathy is characterized by a disorderly growth of heart muscle fibers causing the heart chambers to become thick walled and bulky. The thickening is generally most striking in the walls of the left ventricle, the chamber of the heart which pumps blood through the aorta to the vital organs and tissues of the body. The distorted left ventricle contracts, but the supply of blood to the brain and other vital organs may be inadequate because blood is trapped within the heart during contractions. Restrictive cardiomyopathy causes abnormal cells, proteins, or scar tissue to infiltrate the muscle and structures of the heart, causing the chambers to become stiff and bulky. The heart may initially contract normally, but the rigid chambers restrict the return of blood to the heart.

Massive or multiple heart attacks may also lead to severe heart damage as a result of a disruption of blood supply to heart muscle. The damage can result in functional impairment and structural abnormalities similar to those found in the other types of cardiomyopathy. This type of heart disease, resulting from coronary artery disease, is called *ischemic cardiomyopathy* (*ischemic* meaning "lacking oxygen").

Severe heart injury caused by a major heart attacks or multiple smaller heart attacks may result in heart enlargement and thinning of the chamber walls, abnormalities which resemble those observed in dilated cardiomyopathy. Ischemic cardiomyopathy typically develops in patients with severe coronary artery disease, often complicated by other conditions such as diabetes and hypertension.

Although heart failure symptoms in ischemic cardiomyopathy are similar to those found in dilated cardiomyopathy, ischemic disease is more likely to be accompanied by symptoms of coronary artery disease, such as angina (which is chest pain resulting from reduced oxygen supply to the heart muscle). Diagnosis is typically based on a history of heart attacks and studies that demonstrate poor function in major portions of the left ventricle. The diagnosis can be confirmed by coronary angiography, which reveals areas of narrowing and blockage in the coronary blood vessels.

Patients with ischemic cardiomyopathy are treated with medications that relieve heart failure symptoms and improve blood flow through the diseased coronary arteries, such as nitroglycerin, some types of calcium channel blockers, and angiotensin-converting enzyme (ACE) inhibitors. When symptoms of heart failure and coronary artery disease cannot be controlled with medications, coronary angioplasty or surgery may be considered. Angioplasty and coronary artery bypass grafting may help increase blood flow to the heart, which in turn enhances heart muscle function.

When heart failure symptoms are advanced and cannot be improved by drug therapy or surgery, patients may be referred for a heart transplant. Patients with ischemic cardiomyopathy account for approximately one half of all heart transplant recipients. With a limited supply of donor hearts and complications resulting from heart transplant (such as organ rejection), surgeons have been exploring alternative procedures for treating severe ischemic cardiomyopathy. One such procedure is *transmyocardial revascularization,* otherwise known more simply as "TMR."

TMR procedures revascularize, that is, form new vessels or channels, in the heart muscle or myocardium. The newly formed vessels penetrate through the entire heart wall, which includes the *epicardium* (the outer layer of the heart), the *endocardium* (the inner lining of the heart), and the myocardium or muscular wall therebetween. As ischemic cardiomyopathy more often than not afflicts the left ventricle, the new vessels are typically formed in the heart wall of this chamber of the heart. Accordingly, oxygenated blood from the lungs present in the left ventricle awaiting to be pumped through the aorta is able to flow directly into the newly formed vessels to nourish the heart muscle.

Pioneering methods for performing TMR involved the use of needles for physically puncturing holes in the heart wall. These methods resulted in only a temporary delivery of blood to the myocardium because the holes quickly healed at the endocardium, preventing oxygenated blood from entering the myocardium. One of the more recent and exciting methods of performing TMR is through the use of lasers. It has been observed that new vessels or channels formed in the heart wall by a laser tend to heal at the epicardium, which prevents blood loss, and promote blood perfusion into the ischemic region of the myocardium.

Lasers have proven to be a widely useful and applicable tool in modem medical techniques, particularly in minimally invasive surgical procedures. Technically speaking, a laser (the word *laser* being an acronym for light amplification by stimulated emission of radiation) utilizes the natural oscillations of atoms or molecules between energy levels for generating coherent electromagnetic radiation. A laser is able to produce high-intensity and high-energy light at a single frequency. The energy of laser light is measured in joules (J), or watt-seconds (W-s), and the power of a laser is measured in watts (W).

One of the conventional surgical apparatus for performing TMR consists of a laser and an optical fiber. A surgeon places the end of the optical fiber against the epicardium to ensure that all the laser light is focused at the desired point, and then the laser is fired. In order to form the new vessel completely through the heart wall and into the chamber, the surgeon needs to tactilely urge the optical fiber into and through the epicardium, the myocardium, and the endocardium. Because of the nature of ischemic cardiomyopathy, the thickness of the diseased myocardium is irregular and greater than normal. Accordingly, the surgeon needs to tactilely urge the optical fiber through the heart wall at each location. This procedure takes a certain amount of time to accomplish safely and involves a certain amount of guesswork on the part of the surgeon. This procedure is complicated by the beating of the heart. In addition, irregularly shaped holes may result if the surgeon does not urge the optical fiber into the tissue at a constant rate. For example, a cavity within the new hole may be formed if the surgeon slowed down or paused briefly at a particular location because more tissue at that location would be ablated by the increase in laser energy emitted over time. In addition, the increase in emitted laser energy may cause excessive trauma to the surrounding tissue at that location.

Canadian patent application CA-A-2,201,670 discloses an apparatus for performing a laser myocardial revascularization of a human heart. The apparatus comprises a handheld device with an elongated flexible lasing assembly including an axially movable fiber bundle which can be placed into the chest cavity of a patient.

In many surgical applications, it may be desired to drill as large a hole as possible. For example, in treating an ischemic myocardium, holes with larger diameters have larger inner surface areas; accordingly, more blood is able to perfuse into the ischemic tissue. The difficulty in drilling relatively large holes (for example, about 1 mm) with laser ablation is that the area of the lasing plenum increases exponentially with an increase in the diameter of the hole (the *lasing plenum* being defined as the "bottom" of the hole subject to emitted laser energy). For example, the ratio between the areas of the lasing plenum of a hole with a 0.5-mm diameter and a hole with a 1-mm diameter is four. Conventional practice has been to increase the diameter of the optical fiber and, accordingly, the diameter of the laser beam to form larger holes. The power of the laser may also be increased. However, increasing the diameter of the laser beam results in an increase in the amount of energy emitted and, accordingly, an increase in the trauma of the surrounding tissue. In addition, the power of the laser energy can only be increased to a certain point until the capacity of the optical fiber is exceeded.

Accordingly, in view of the foregoing, it is an object of the present invention to provide methods and associated apparatus for forming holes or channels in tissue in a consistent and controlled manner.

It is another object of the present invention to provide surgical apparatus for forming holes or channels in tissue with a tissue drill using laser ablation.

It is a further object of the invention to provide surgical apparatus and method for forming holes in tissue in a substantially reduced traumatic manner.

It is yet another object of the present invention to provide methods and associated apparatus for performing transmyocardial revascularization.

### SUMMARY OF THE INVENTION

These and other objects are achieved by the surgical apparatus and associated methods of the present invention which provides a tissue drill for forming holes or channels in tissue by laser ablation in a controlled and consistent manner, as defined in claim 1. In a broad aspect, the tissue drill for reduced-trauma tissue ablation includes.an optical fiber and a handpiece. The optical fiber has an inlet for receiving laser energy from a laser energy source and an outlet for emitting laser energy. The handpiece is adapted to receive the optical fiber in a controlled and movable relationship. Exemplary handpieces can include flexible catheters, biocompatible tubular members, trocar sheaths, and the like. In use, a distal end of the handpiece is placed near or against tissue to be ablated. The optical fiber is advanced beyond the distal end of the handpiece and into the tissue while emitting laser energy. The emitted laser energy ablates the tissue as the optical fiber advances. The optical fiber may then be removed from the tissue, thereby resulting in a channel formed in the tissue. Although capable of performing all types of tissue drilling, the tissue drill of the present invention is particularly suitable for performing transmyocardial revascularization (TMR), which is the forming of channels in or through the wall of the heart.

The tissue drill of the present invention controls this tissue-ablating procedure. For example, the distance at which the optical fiber advances into the tissue may be predetermined and varied. Also, the emission of laser energy may be terminated as soon as the optical fiber has advanced the predetermined distance. In addition, the speed at which the optical fiber is advanced into the tissue may also be controlled and varied. This controllable and variable nature of the tissue drill enables a physician to program the tissue drill according to a particular procedure and a particular patient. It also provides a consistent procedure which allows multiple holes to be identically formed in tissue. By enabling a physician to control the drilling process in this manner, guesswork is substantially eliminated from the operation.

According to another aspect of the present invention, the handpiece of the tissue drill may be adapted to receive the optical fiber in a rotatable relationship. In operation, while advancing into the tissue, the optical fiber may rotate while emitting laser energy. In this regard, the outlet may be eccentrically positioned with respect to the axis of rotation of the optical fiber. As such, the outlet rotates about the axis of rotation. This eccentric relationship results in laser energy being emitted and focused about the axis of rotation, which is essentially at a center of a hole being formed, and emitted at a lower level at a periphery of the rotating optical fiber, which is adjacent to tissue surrounding the hole being formed. Accordingly, a lower level of laser energy is incident on the surrounding tissue, thereby reducing trauma to the tissue.

The handpiece of the present invention includes a body portion and a coupling portion for receiving the optical fiber in controlled and axially movable relationship with respect to the body portion. The coupling portion includes a drive for moving the outlet of the optical fiber to a position beyond the distal end of the handpiece. The drive may also move the outlet to a position substantially at or near the distal end. Accordingly, the drive may reciprocate the outlet of the optical fiber between these advanced and retracted positions. The coupling portion may also be adapted to receive the optical fiber in a rotatable relationship. Alternatively, the handpiece may be a flexible tubular member such as a catheter with a drive and coupling portion positioned at a proximal end or on external equipment. In this regard, endovascular surgical procedures may be undertaken, such as performing transmyocardial revascularization from the inside of the left ventricle.

The optical fiber of the present invention may include an elongate portion and an outlet portion. A core of the elongate portion has an inlet for receiving laser energy, and a core of the outlet portion has an outlet for emitting laser energy. An axis of the core of the outlet portion may be oblique to an axis of the core of the elongate portion. This oblique relationship results in the eccentric position of the outlet with respect to the axis of rotation of the optical fiber discussed above. In addition, the outlet portion may have an end surface from which the outlet emits laser energy. To provide the differing levels of laser energy emitted from the end surface as optical fiber rotates, the outlet has a diameter of at least approximately one half of a diameter of the end surface. Accordingly, a surface area of the outlet is at least one quarter of the surface area of the end surface. This relationship between diameters and surface areas results in an emission of laser energy focused about the axis of rotation and an intermittent emission of laser energy at the annular areas or the periphery of the end surface. Accordingly, tissue adjacent to the periphery of the end surface as the outlet portion advances is subject to a relatively low level of laser energy. This results in less trauma to the tissue which defines the inner surface of the newly formed channel. It has been observed that low-traumatized tissue has a likelihood to regenerate vascular tissue. Such revascularization or *angiogenesis* promotes the healing of the ischemic tissue.

Other aspects, features, and advantages of the present invention will become apparent to those persons having ordinary skill in the art to which the present invention pertains from the following description taken in conjunction with the accompanying drawings.

### BRIEF DESCRIPTION OF THE DRAWINGS

FIG. **1** is a perspective view of an exemplary embodiment of a tissue drill of the present invention;
FIG. **1A** is a cross-sectional view of an exemplary optical fiber of the invention taken along line 1A of FIG. **1**;
FIG. **2A** is a diagrammatic view of the exemplary tissue drill of the present invention, illustrating a handpiece receiving an optical fiber in a retracted position;
FIG. **2B** is a diagrammatic view similar to that of FIG. **2A**, illustrating the optical fiber in an advanced position;
FIG. **3A** is a diagrammatic view of an exemplary handpiece of the tissue drill of the present invention, illustrating the handpiece disassembled;
FIG. **3B** is a diagrammatic view similar to that of FIG. **3A**, illustrating the handpiece assembled;
FIG. **4** is a schematic view of an exemplary optical fiber of the present invention, particularly illustrating an eccentric configuration of an outlet portion of the optical fiber;
FIG. **5** is a schematic view of an end surface of the optical fiber illustrated in FIG. **4**;
FIG. **6** is a schematic view of another exemplary optical fiber of the present invention;
FIG. **7** is a schematic view of an end surface of the optical fiber illustrated in FIG. **6**;
FIG. **8** is a diagrammatic view of an exemplary end surface of an optical fiber of the present invention, particularly illustrating a relationship between emitted laser energy and position of the end surface;
FIG. **9** is a schematic view of an exemplary source of laser energy of the present invention;
**FIG. 10A** is a schematic view of an exemplary tissue drill of the present invention, particularly illustrating a step of a preferred tissue-drilling procedure implementing the tissue drill;
**FIG. 10B** is a view similar to that of **FIG. 10A****,** illustrating a subsequent step in the tissue-drilling procedure;
**FIG. 10C** is a view similar to that of FIG. **10B**, illustrating another subsequent step in the tissue-drilling procedure;
**FIG. 10D** is a view similar to that of FIG. **10C**, illustrating yet another subsequent step in the tissue-drilling procedure;
FIG. **11** is a schematic view of tissue in which a hole has been drilled according to an exemplary method of the invention; and
FIG. **12** is a schematic view of tissue in which a hole has been drilled according to another exemplary method of the invention.

### DETAILED DESCRIPTION OF EXEMPLARY EMBODIMENTS

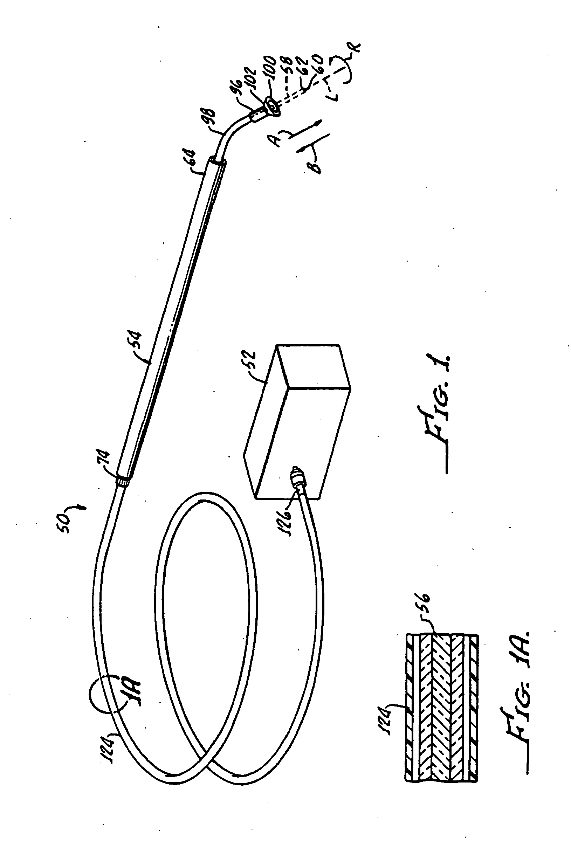

Referring to the drawings in more detail, in FIG. 1 an exemplary embodiment of a tissue drill **50** of the present invention is illustrated in conjunction with a source of laser energy **52**. Exemplary tissue drill **50** forms holes or channels in tissue by laser ablation in a consistent, controllable, and programmable manner. Ablation is the process of fragmenting long molecules into short gaseous molecules. Much of the tissue in living organisms, including the human body, is made up mostly of water (e.g., about 75%) with organic material making up the remaining portion. The molecules of organic material consist of atoms of carbon, nitrogen, oxygen, and hydrogen that are attached together through covalent bonds. Ablation is the process of breaking these covalent bonds. Tissue drill **50** utilizes the ablation process to break molecules of tissue apart, thereby forming holes or channels in the tissue. The ablation process will be discuss in more detail below.

Exemplary tissue drill 50 includes a handpiece **54** for manipulation by a user and an optical fiber **56**, which is shown in FIG. **1A**, for transmitting laser energy from laser energy source **52**. Optical fiber **56** has an outlet portion **58** for emitting laser energy. Outlet portion **58** functions substantially as a drill bit. In operation, outlet portion **58** is moved from a retracted position (which is shown in the solid line) to an advanced position (which is shown by the phantom line) while emitting laser energy. Arrow A represents outlet portion **58** moving to the advanced portion, and arrow L represents laser energy emitted from outlet portion **58.** Tissue is ablated by laser energy as outlet portion **58** is advanced, thereby forming a hole or a channel in the tissue. Exemplary tissue drill **50** may also rotate outlet portion **58** while moving to the advanced position, which is represented by arrow R. After reaching the advanced position, outlet portion **58** may be withdrawn to the retracted position, which is represented by arrow B. The advancing and retracting of outlet portion **58** is preferably along a central axis of optical fiber **56**. Any rotation of outlet portion **58** is preferably about the central axis of optical fiber **56.** The axial and rotational movement of outlet portion **58** will be discussed in more detail below.

Exemplary outlet portion **58** of optical fiber **56** has an end surface **60** with an outlet **62** from which laser energy is emitted. Outlet **62** is preferably offset from or eccentric to the central axis of outlet portion **58** so that as outlet portion **58** rotates, outlet **62** rotates about the central axis. Accordingly, laser energy emitted from outlet **62** as outlet portion **58** rotates is not focused at a single point but is rather distributed about the central axis. Alternatively speaking, the eccentric relationship of outlet **62** with respect to the central axis of outlet portion **58** preferably produces a gradient of laser energy as outlet portion **58** axially advances, with the highest level of laser energy at the central axis, which energy decreases toward a peripheral edge. The eccentricity of outlet portion **58** will also be discussed in more detail below.

Handpiece **54** may be implemented according to a variety of configurations. For example, handpiece **54** may be a flexible catheter utilized in endovascular procedures in conjunction with a camera. In this regard, outlet portion **58** may advance beyond a distal end of the catheter to vascularize tissue, such as on the inside the left ventricle of the heart. Alternatively, handpiece **54** may be formed as a trocar sheath and positioned intercostally (i.e., between the ribs) for tissue access. Handpiece **54** may also be formed in a gooseneck-like configuration with a plurality of articulated joints which may be bent to assume and retain a particular shape. Moreover, handpiece **54** may be a conduit with flexible cable sheathing. Accordingly, in a general sense, handpiece **54** provides a "user interface" for delivering outlet portion **58** to a target site, which may be accomplished either by direct physical manipulation by a surgeon or by programmed mechanical control.

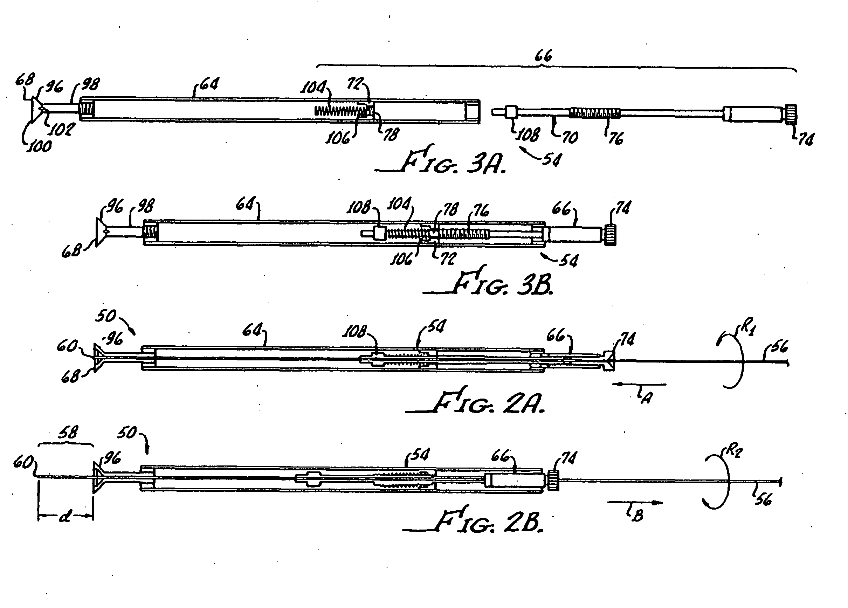

An exemplary handpiece of the present invention is illustrated in FIGS. **2A** and **2B****.** Exemplary handpiece **54** may include a body portion **64** and a coupling portion **66.** Exemplary body portion **64** has a distal end **68.** Exemplary coupling portion **66** is adapted or configured to receive optical fiber **56** in a controlled and axially movable relationship so that outlet portion **58** may be advanced beyond distal end **68** of body portion **64.** In addition, coupling portion **66** may be adapted to receive optical fiber **56** in a rotatable relationship so that at least outlet portion **58** of optical fiber **56** may rotate. If handpiece **54** is configured as a catheter or similar flexible tubular member, the inner surface of the tubular member serves as a coupling portion by receiving optical fiber **56** in a controlled, axially movable, and/or rotatable relationship.

The retracted position of outlet portion **58** as shown in FIG. **2A** may be defined as a position in which end surface **60** is positioned substantially at or near distal end **68** of body portion **64.** Accordingly, end surface **60** may project slightly beyond distal end **68** or, alternatively, may be either proximal to or substantially aligned (or coplanar) with distal end **68.** The advanced position of outlet portion **58** as shown in FIG. **2B** may be defined as a position in which end surface **60** with outlet **62** projects a distance *d* beyond distal end **68** of body portion **64.** As will be discussed in more detail below, distance *d* at which end surface **60** projects beyond distal end **68** is preferably predetermined, adjustable, and/or programmable.

With additional reference to FIGS. **3A** and **3B**, exemplary coupling portion **66** may include a drive which is comprised of a tubular member **70** and a collar **72.** Tubular member **72** receives optical fiber **56** and may have a chuck **74** for retaining optical fiber **56** thereto. Tubular member **72** may also have annular threading **76** formed along a length thereof. Collar **72** is disposed within body portion **64** and has complementary inner threading **78.** Exemplary tubular member **72** is slidably and rotatably receivable within body portion **64** with annular threading **76** engaging with inner threading **78** of collar **72,** as shown in FIG. **3B**. Accordingly, rotation of tubular member **70** causes tubular member **70** to move axially. As optical fiber **56** is retained by chuck **74**, optical fiber **56** with outlet portion **58** moves axially with tubular member **70.** In an alternative embodiment of handpiece **54** such as a catheter, rather than disposing coupling portion **66** and a drive on handpiece **54,** these elements may be provided at a proximal location, such as at laser apparatus **52.** In this regard, catheter-configured handpiece **54** retains optical fiber **56** within a body portion which prevents buckling and which delivers outlet portion **58** to a target site but which is substantially free of coupling and drive apparatus.

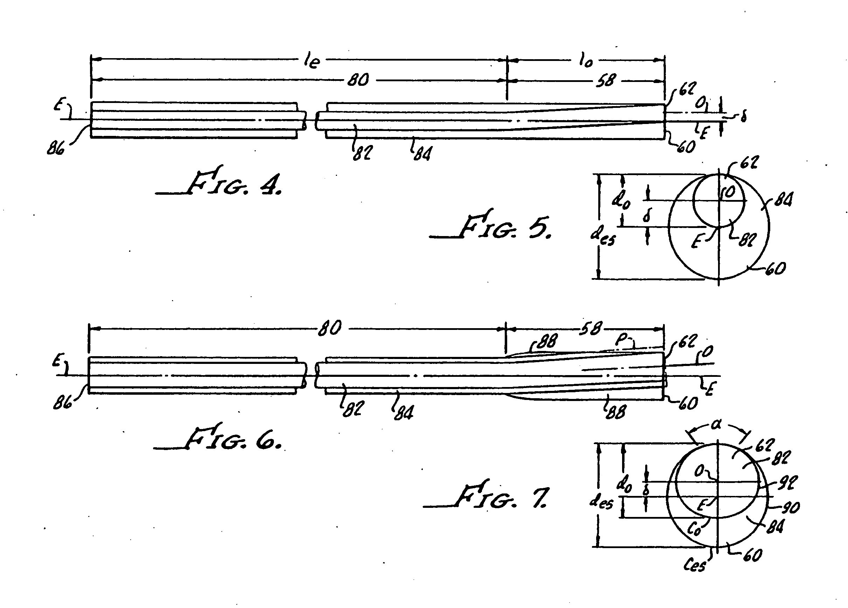

Referencing FIG. **4****,** in addition to outlet portion **58,** exemplary optical fiber **56** has an elongate portion **80.** A core **82** and a cladding **84** define optical fiber **56** and extend along elongate portion **80** and outlet portion **58.** Core **82** has an inlet **86** for receiving laser energy and outlet **62** (see also FIG. **1**) for emitting laser energy. Core **82** and cladding **84** may be made of high-purity silica glass or sapphire, with core **82** having a higher index of refraction than that of cladding **84** so that modulated pulses of laser energy move along core **82** without penetrating cladding **84.** Although optical fiber **56** may be configured according to any dimensions, for many applications a length lₑ of elongate portion **80** may range from about 0.5 meter (m) to more than 2 m to provide a surgeon with sufficient maneuverability, and a length lₒ of outlet portion **58** may range up to about 50 millimeters (mm) so that holes of different lengths may be formed in tissue. For applications other than medical, optical fiber **56** may be dimensioned accordingly to accomplish the particular application.

Core **82** of optical fiber **56** has an axis E along elongate portion **80** and an axis O at outlet **62.** With additional reference to FIG. **5****,** core **82** along outlet portion **58** angles away from and is oblique to core **82** along elongate portion **80.** At end surface **60,** axis O of core **82** at outlet **62** is offset from or eccentric to axis E of core **82** of elongate portion **80** by a distance δ. Accordingly, laser energy emitted from outlet **62** is distributed about axis E as optical fiber **56** rotates about axis of rotation E. Further, the distribution of laser energy is across the entire surface area of end surface **60** as optical fiber **56** make one complete revolution. At end surface **60,** outlet **62** may be configured so that axis O of core **82** is either oblique to axis E or, as shown, parallel to axis E.

An alternative exemplary embodiment of optical fiber **56** is illustrated in FIGS. **6** and **7****.** In addition to core **82** and cladding **84,** exemplary optical fiber 56 may include auxiliary cladding **88** disposed about outlet portion **58.** Similar to the embodiment shown in FIG. **4****,** to offset axis O of outlet **62** from axis of rotation E by distance δ, core **82** of outlet portion **58** is oblique to core **82** of elongate portion **80.** Auxiliary cladding **88** compensates for the oblique relationship of core **82** (and cladding **84**) of outlet portion **58** with respect to core **82** (and cladding **84**) of elongate portion **80.** Auxiliary cladding **88** accordingly provides a preferred cylindrical configuration of outlet portion **58** so that outlet portion **58** rotates about axis E as elongate portion **80** rotates about axis E. Further, in addition to axis O at outlet **62** being eccentric to axis E, axis O of core **82** may be oblique to axis E at outlet **62,** rather than a parallel relationship as shown in FIG. **4****.**

As illustrated in FIGS. **6** and **7****,** end surface **60** (including outlet **62**) is substantially perpendicular to axis E of exemplary optical fiber **56.** To form the perpendicular relationship, core **82** and cladding **84** are ground or polished at an angle oblique to axis O, thereby removing portions of core **82** and cladding **84** shown by phantom line P. Accordingly, exemplary end surface **60** is substantially planar. Alternatively, end surface **60** may be convex, concave, or other configuration depending upon a particular implementation of outlet portion **58.**

With particular reference to FIG. **7****,** end surface **60** of exemplary optical fiber **56** has a circumference Cₑₛ defined along an outer edge **90,** and outlet **62** of core **82** has a circumference Cₒ defined along outer edge **92.** Circumference Cₑₛ and circumference Cₒ are coextensive along an arc length α of outer edges **90** and **92**. This relationship allows laser energy to be emitted from outlet **62** at outer edge **90** of end surface **60**. As outlet portion **58** rotates, laser energy is emitted along circumference Cₑₛ of rotating end surface **60.** Arc length α may range from a single tangent point to several seconds, minutes, or degrees as desired.

Diameter dₒ of outlet **62** is preferably greater than about one half of diameter dₑₛ of end surface **60.** Accordingly, outlet **62** has a surface area which is at least one quarter of that of end surface **60.** This relationship in surface area allows laser energy to be emitted from a substantial percentage of end surface **60.** Further, laser energy is not emitted from the entire end surface **60** simultaneously but rather over the time it takes outlet portion **58** to make one revolution about axis E. An exemplary commercial embodiment of optical fiber **56** for use in transmyocardial revascularization entails a diameter des of end surface **60** (and outlet portion **58** of approximately 1 mm and a diameter do of outlet **62** of approximately 0.6 mm. Generally speaking, the dimensions of outlet portion **58** are determined by the type of procedure being performed and the desired size of the hole, with diameter do of outlet **62** being at least one half of diameter dₑₛ of end surface **60.** For example, if a hole with a 1.5-mm diameter is desired, then diameter dₑₛ of end surface **60** (and outlet portion **58**) should be about 1.5 mm; diameter do of outlet **62** may accordingly range from about 0.75 mm to slightly less than 1.5 mm, but is preferably about 0.8 mm. For many medical applications, it is contemplated that diameter dₑₛ of end surface **60** may range from about 0.2 mm to more than 2.5 mm, with diameter do of outlet **62** ranging from less than about 0.1 mm to about 2 mm or more. For specific medical applications such as transmyocardial revascularization (which will be discussed below), diameter dₑₛ of end surface **60** may range from about 0.6 mm to about 2 mm, with diameter dₒ of outlet **62** ranging from about 0.3 mm to about 1 mm.

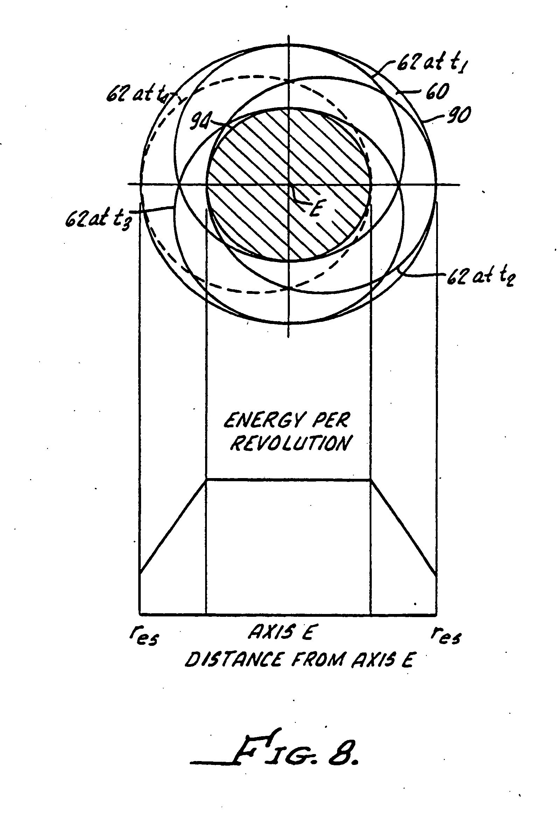

With additional reference to FIG. **8****,** end surface **60** is schematically illustrated during rotation, with outlet **62** shown at progressive instances in time *t₁, t₂, t₃,* and *t₄* while rotating about axis E. Because of the relationship between the surface areas of end surface **60** and outlet **62,** laser energy is continuously emitted from an area **94** of end surface **60.** In other words, area **94** represents an intersection of the positions of outlet **62** at every instance of time while rotating about axis E. Laser energy is accordingly emitted at intervals at other areas of end surface **60** depending upon the position of outlet **62** at a particular instance in time.

The relationship between laser energy emitted from exemplary end surface **60** per revolution of outlet **62** about axis E with respect to distance from axis E is illustrated graphically in FIG. **8****.** Emitted laser energy per revolution of outlet portion **58** decreases from a constant level at area **94** to a lower level at outer edge **90** of end surface **60.** In the graph, outer edge **90** is a distance from axis E substantially equal to radius rₑₛ of end surface **60.** Depending upon a particular configuration of exemplary end surface **60** and outlet **62,** the decrease in laser energy or flux with respect to position may be a linear function as shown or a nonlinear function. Also, the relative level of energy per revolution at area **94** and at radius rₑₛ is illustrative only, as the level of energy at the periphery of end surface **60** may vary according to the particular surgical procedure. For example, the energy flux at radius rₑₛ may be at a relatively low level when compared to the constant level at area **94.**

In accordance with this energy distribution per revolution of the present invention, while ablating tissue to form a hole, the transference of laser energy to peripheral or surrounding tissue is less than at a center of the hole being formed. This distribution of laser energy may limit trauma to tissue in which holes or channels are formed. More specifically, as outlet portion **58** moves through tissue while rotating and emitting laser energy, outer edge **90** of end surface **60** is adjacent to and contacts the surrounding tissue which defines the hole being formed. As the level of emitted laser energy at outer edge **90** is lower than that centered about axis E (which essentially defines the center of the hole being formed), damage to the surrounding tissue is reduced, resulting in less trauma to the tissue. It is believed that tissue with a relatively low level of trauma has a likelihood to experience angiogenesis, or the formation of new blood vessels in the tissue. This reduced-trauma feature of the present invention will be discussed in more detail below.

An exemplary process to form an eccentric outlet portion **58** as described above involves placing the distal end of optical fiber **56** within a Teflon^{®} tube at an angle, with cladding **84** contacting the inner surface of the tube at one point. The tube may then be filled with epoxy which surrounds the distal end of optical fiber **56** except at the point at which cladding **84** contacts the tube. After the epoxy has cured and hardened, the tube is removed, and the distal surface of the epoxy and optical fiber **56** is polished to define end surface **60** at the point where cladding **84** defines an annular edge of outlet portion **58.** End surface **60** may also be formed with a lens to control the emission of the laser energy in a particular manner. An inner diameter of the tube for forming outlet portion **58** essentially determines the diameter of outlet portion **58** (i.e., diameter dₑₛ of end surface **60**). According to this process, optical fibers **56** having outlet portions **58** of different diameters may be formed, enabling surgeons to form holes with a variety of diameters. In addition, a plurality of outlet portions **58** each having a different diameter may be formed, each of which being able to be coupled to an optical fiber, so that a set of interchangeable "drill bits" is at a surgeons disposal during a particular procedure. Optical fiber **56** may be reusable or disposable, as may outlet portion **58** and handpiece **54.**

With further reference to FIGS. **1** and **3A****,** exemplary of handpiece **54** may include a head portion **96** connectable to a distal end of body portion **64** by a neck **98.** Distal end 68 of body portion **64** is accordingly defined by a tissue end **100** of head portion 96. Exemplary head portion **96** may be conical so that tissue end **100** has a larger diameter than body portion **64.** Tissue end **100** provides a working surface or a tissue-engaging surface for positioning handpiece **54** over and against a surgical site in which a channel is to be drilled into tissue. Exemplary head portion **96** may also have an aperture **102** formed therein. Aperture **102** may function as a window for viewing a surgical site when tissue end **100** is placed against tissue. Aperture **102** may also function as a vent for exhausting gases which may be generated by laser energy ablating tissue. As shown in FIG. 1, exemplary neck **98** may be angular to enhance the positioning of head portion **96** against tissue. In this regard, neck **98** may be configured as a gooseneck with articulable joints for assuming and retaining a desired shape. Exemplary head portion **96** and neck **98** are preferably tubular, thereby providing an inner continuum with body portion **64** in which optical fiber **56** is receivable.

In particular procedures, it may be preferable to know where a hole has been drilled in tissue. However, the nature of the tissue or the size of the hole may render it difficult for the surgeon to determine where a hole has already been formed. Accordingly, the newly formed hole drilled in tissue may be marked. In this regard, head portion **96** may include apparatus for marking where a hole has been drilled in tissue. For example, tissue end **100** may have an inking device which dispenses biocompatible ink or dye on the tissue where a hole has been formed. The ink may be applied to the tissue through direct contact with tissue end **100** or, for example, by spraying. Exemplary handpiece **54** may have a reservoir for storing and dispensing a colored liquid or a particulate solid to the tissue. Fluorescent material may be used to enhance visualization. Other indicia may be applied to the tissue by handpiece **54** or head portion 96 at the target site; for example, alphanumeric indicia may indicate the parameters of the laser energy emitted from outlet **62** to form a particular hole.

With further reference to FIGS. **2A** to **3B****,** exemplary coupling portion **66** may include a spring **104** receivable against a seat **106** formed on a distal end of collar **72,** and a stop **108** disposed on a distal portion of tubular member 70. Spring **104** and stop **108** define a mechanism for controlling a position of tubular member **70** within body portion **64,** and may be configured to facilitate the advancement and retraction of tubular member **70.**

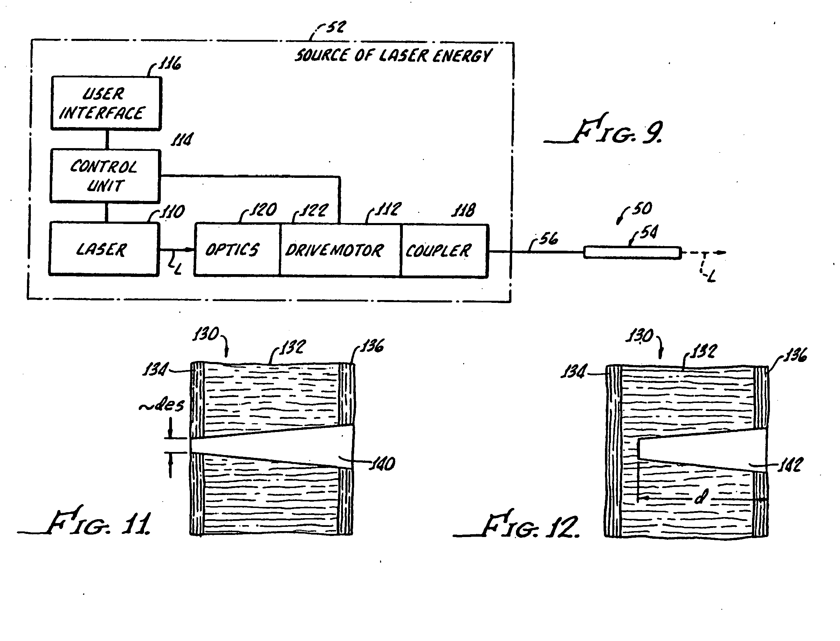

Exemplary source of laser energy **52** is illustrated in FIG. **9****.** Laser energy source **52** includes a laser **110** for generating laser energy L. Exemplary laser energy source **52** may include a drive assembly **112** for operatively associating with handpiece 54 and optical fiber **56,** and may also include a control unit **114** with a user interface **116.** Exemplary drive assembly **112** may include a coupler **118** for connecting with optical fiber 56, optics **120** for modifying laser energy L as desired, and a drive/motor **122.** Exemplary coupler **118** is associated with optics **120** for transferring laser energy L from laser **110** to the inlet of optical fiber **56.** Exemplary coupler **118** is also associated with drive/motor **120** for rotating optical fiber **56.**

As discussed above in reference to FIGS. **2A** and **2B**, exemplary coupling portion **66** of handpiece **54** translates rotational movement of optical fiber **56** to axial movement to advance and to retract outlet portion **58.** Exemplary drive assembly **112** preferably rotates optical fiber **56.** For example, coupler **118** may secure and retain a proximal end of optical fiber **56,** with motor/drive **122** rotating coupler **118** which also rotates optical fiber **56.** Drive assembly **112** may rotate optical fiber **56** in a first direction, for example, as shown by arrow R₁ in FIG. **2A****,** to cause optical fiber **56** to advance axially as shown by arrow A. When outlet portion **58** reaches the desired advanced position, drive assembly **112** may then rotate optical fiber **56** in an opposite second direction, as shown by arrow R₂ is FIG. 2B, to cause optical fiber **56** to retract axially as shown by arrow B. Exemplary drive assembly **112** may oscillate optical fiber **56** (that is, rotate optical fiber **56** clockwise and counterclockwise as shown by arrows R, and R₂) so that outlet portion **58** reciprocates between the retracted position and the advance position.

Exemplary laser energy source **52** preferably controls when laser energy L is emitted from outlet portion **58** of optical fiber **56.** For example, control unit **114** in association with laser **110** and drive assembly **112** may limit the emission of laser energy L to only when outlet portion **58** moves to the advance position. Laser energy L may then be terminated during the retraction of outlet portion **58.** Alternatively, if drive assembly **112** is reciprocating outlet portion **58,** laser energy L may be transmitted only during the advancing stroke of outlet portion **58;** the emission of laser energy L may then be terminated at the end of the advancing stroke. The termination of laser energy L upon reaching the advanced position is preferably automatic and controlled by laser energy source **52.** Alternatively, laser energy L may be terminated by a device such as a pressure sensor which determines when the distal end of outlet portion **58** advanced completely through a section of tissue, e.g., the wall of the heart. This control of laser energy L is preferable during particular applications of tissue drill 50, which will be discussed in more detail below.

With further reference to FIG. **1A****,** optical fiber **56** is preferably received within a housing **124.** In addition to protecting optical fiber **56,** exemplary housing **124** constrains any torsional flexing or bending of optical fiber **56** which may result from the rotation by drive assembly **112.** Exemplary optical fiber **56** may include a complementary coupler **126** for connecting with coupler **118** of laser energy source **52**. Complementary coupler **126** preferably provides a releasable association with coupler **118** so that other optical fibers in accordance with the present invention may be connected to laser energy source **52.** Exemplary housing **124** preferably extends between coupler **126** and chuck **74** of coupling portion **66** to provide integral protection of optical fiber **56** between laser energy source **52** and handpiece **54**.

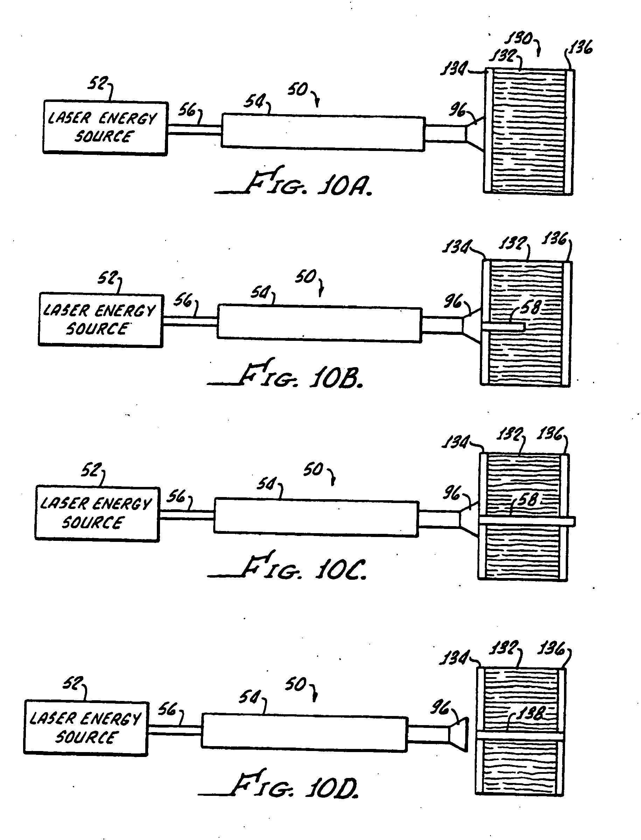

Exemplary laser energy source **52** may control a number of parameters of tissue drill **50,** including distance *d* at which outlet portion **56** advances, a speed at which outlet portion 56 advances, and a level at which laser energy is emitted from outlet **62.** Control unit **114** in association with user interface **116** preferably controls, programs, monitors, and/or adjusts each of these parameters. depending upon a particular tissue-drilling application. For example, one the many applications of tissue drill **50** is for drilling holes or channels into or through heart walls. This procedure is known as *transmyocardial revascularization* or, more simply, as TMR. FIGS. **10A** through **10D** schematically illustrate an exemplary TMR procedure implementing tissue drill **50** of the present invention.

A heart wall **130** is illustrated in FIG. **10A** and includes myocardium, or heart muscle, **132** positioned between an outer serous layer or epicardium **134** and an inner membrane or endocardium **136.** It has been found to be medically beneficial to revascularize the myocardium of patients suffering from severe ischemic cardiomyopathy. The revascularization of the myocardium **132** involves forming new channels in the tissue. By implementing exemplary tissue drill **50** of the present invention, new channel may be formed in the myocardium in a controlled, consistent, and programmable manner.

Prior to a TMR procedure, the level at which laser **110** is to generate laser energy L and the frequency at which laser energy L is to be pulsed may be determined. In addition, distance *d* at which outlet portion **58** is to advance beyond distal end **68** and the speed at which outlet portion **58** is to rotate may be determined. These parameters may be stored in control unit **114** and varied or programmed via user interface **116.**

During the TMR procedure, access to the patient's chest cavity is provided, preferably by a minimally invasive procedure such as an intercostal incision using trocar sheaths. Access to the patient's heart is then provided, for example, by incising the pericardium. With outlet portion **58** in the retracted position, a surgeon may then maneuver head portion **96** of handpiece **54** into the chest cavity and position tissue surface **100** against the epicardium **134,** as shown in FIG. **10A****.** As discussed above, outlet portion **58** may project slightly beyond distal end **68** (that is, tissue end **100**) when in the retracted position to provide the surgeon with a tactile feel of the position of end surface **60** on the epicardium **134.**

When in the desired position on the epicardium **134,** tissue drill **50** may be activated. This activation may be accomplished manually by an assistant via user interface **116** or by the surgeon with a foot or a hand trigger. Alternatively, activation of tissue drill **50** may be synchronized with the electrical activity of the heart through the use of an electrocardiogram (EKG) machine. Activation of tissue drill **50** causes laser energy source **52** to generate and transmit laser energy to optical fiber **56.** Activation also causes optical fiber **56** to rotate and advance outlet portion **58** through the epicardium **134** and into the myocardium **132** of the heart wall **130**, as shown in FIG. **10B****.**

Outlet portion **58** continues to advance through the myocardium **132** and through the endocardium **136.** When end surface **60** of outlet portion **58** has advanced through the endocardium **136** and is positioned within the left ventricle of the patient's heart as shown in FIG. **10C****,** the emission of laser energy is preferably terminated, and the outlet portion **58** is retracted. A new channel **138** through the heart wall **130** results from this procedure as shown in FIG. **10D****.** Oxygenated blood from the left ventricle may enter the new channel **138** through the endocardium **136** and perfuse the tissue of the myocardium **132** surrounding the new channel **138.** When handpiece **54** is configured as a catheter, outlet portion **58** advances through the endocardium **136** and then into the myocardium **132.** Because outlet portion **58** may be programmed to advance a predetermined distance, outlet portion **58** may either continue to advance completely through the epicardium **134** or begin to retract the predetermined distance within the myocardium **132,** thereby forming a hole in the heart wall **130** rather than a channel through the heart wall **130.**

As mentioned above, reduced trauma to the myocardium **134** surrounding the new channel **138** results from the eccentric relationship between outlet **62** and rotational axis E. This reduced trauma may enable the surrounding tissue to regenerate vascular tissue from the new channel **138** and into the myocardium **134** or to experience angiogenesis. In addition to the eccentricity of outlet portion **58,** the level of trauma inflicted on the surrounding tissue is mediated by the level of laser energy emitted from outlet **62,** which will now be discussed.

With reference to FIG. **9****,** the energy level at which laser energy L is generated and transmitted to optical fiber **56** may be varied, programmed, and controlled according to each tissue-drilling application. For example, tissue drill **50** may be configured for drilling holes in all types of animal tissue and plant tissue, as well as other substances. The parameters which define the characteristics of laser ablation include frequency, energy per channel, pulse width, and pulse rate. As mentioned earlier, ablation is a process of breaking bonds between atoms in molecules by adding energy to the molecules. One preferred level of the laser energy L for TMR applications is to limit the energy per pulse to less than about 100 milliJoules per square millimeter of area (mJ/mm²). More preferably; an energy per pulse of about 30 mJ/mm² has been found to ablate cardiac tissue at a substantially reduced level of trauma. The energy per pulse of laser **110** may be varied according to specific tissue-drilling procedures.

With further reference to FIGS. **3A** and **3B**, the drive may be configured to control the rate at which outlet portion **58** advances and retracts. The rate of advancement is controlled by the speed at which optical fiber 56 rotates and the pitch of the complementary threading of collar **72** and tubular member **78.** For smooth and continuous operation, it has been determined that optical fiber **56** and, accordingly, outlet portion **58** should rotate at a speed under about 5,000 revolutions per minute (RPM). For TMR applications of tissue drill **50,** a rotational speed ranging from about 1,000 RPM to about 2,000 RPM is preferred. In this regard, a specific TMR configuration of tissue drill **50** may be as follows. Optical fiber **56** may rotate at about 1,340 RPM. The pitch of threading **76** and **78** may be configured so that outlet portion **58** advances at a rate of about 15.5 millimeters per second (mm/s). With a rotational speed of 1,340 RPM, it takes about 46 milliseconds (ms) for outlet portion **58** to complete one rotation. For TMR applications, laser **110** may emit pulses of laser energy L of about 20 nanoseconds (ns) in duration, with each pulse being separated by about 4 ms. The pulse rate may be about 10 pulses per revolution (or at about every 36° of rotation) or about 240 pulses per second.

Rather than advancing and retracting outlet portion **58** at a constant rate as described above, tissue drill **50** may be configured such that outlet portion **58** moves at varying rates of speed between the retracted and advanced positions. The slower outlet portion **58** advances (or retracts) while emitting laser energy L, the more tissue that becomes ablated because the tissue is subject to more laser energy over time. Accordingly, a hole may be formed with a diameter greater than diameter dₑₛ of end surface 60 (and outlet portion **58**) by advancing outlet portion **58** at a speed which allows laser energy L to ablate a greater amount of tissue. Alternatively, the power of laser energy L may also be varied during the advancement of outlet portion **58** so that the tissue is subjected to more or less laser energy L. Generally speaking, a surgeon may program tissue drill **50** to ablate tissue at varying levels of energy per unit time to form holes of varying desired diameters or configurations. The energy per unit time may be adjusted by varying either the speed at which outlet portion **58** advances (which varies the time the tissue is subject to laser energy) or the level of laser energy, or both.

In order to form the substantially cylindrical hole **138** shown in FIG. **10D****,** tissue drill **50** advanced outlet portion **58** at a substantially constant speed, and laser energy source **52** emitted laser energy at a substantially constant level. However, if a conical-shaped hole **140** as shown in FIG. **11** is desired, with the apex of the hole **140** positioned at the epicardium **134** and the base of the hole **140** positioned at the endocardium **136,** then tissue drill **50** may be configured to advance outlet portion **58** at a decreasing rate (i.e., moving at a slower and slower speed) while advancing through the heart wall **130** from the epicardium **134** to the endocardium **136.** Accordingly, a greater amount of tissue is ablated as outlet portion **58** advances at a slower speed. The resulting hole **140** has a diameter substantially equal to diameter dₑₛ of outlet portion **58** at the epicardium **134** and a diameter larger than diameter dₑₛ at the endocardium **136.** By forming the hole **140** with a relatively large diameter at the endocardium **136** improves the patency of the hole and, therefore, the perfusion of the blood into the myocardium **132.** In addition, by forming a hole with as small a diameter as possible at the epicardium **134** minimizes bleeding and trauma.

With reference to FIG. **12****,** another noncylindrically shaped hole **142** is shown. Rather than forming hole **142** by advancing outlet portion **58** from the epicardium **134 to** the endocardium **136** as shown in FIG. **11****,** hole **142** is formed endovascularly, with outlet portion **58** advancing from the endocardium **136** and into the myocardium **132** a predetermined distance *d*. As mentioned above, to form holes endovascularly, handpiece **54** may be configured as a catheter, with access to the left ventricle of the heart provided through, for example, a femoral artery and the aorta. To form hole **142** with a diameter greater than diameter dₑₛ of end surface **60** at the endocardium **136,** tissue drill **50** is configured to advance outlet portion 58 relatively slowly at or near the epicardium **136** and then to increase the speed. This results in more tissue being ablated at or near the endocardium **136** than at the "bottom" of hole **142** within the myocardium **132.** Laser energy may also be emitted while outlet portion **58** retracts to ablate more tissue toward the endocardium **136.** The speed of advancement may be varied by varying either the revolutions per second at which outlet portion **58** rotates or the pitch of threading **76** and/or **78,** or both. As mentioned above, rather than varying the speed at which outlet portion **58** advances, the level of emitted laser energy L may be varied. In this regard, to form hole **142,** tissue drill **50** may be configured to emit laser energy L at a relatively high level when outlet portion **58** begins to advance, and then to decrease the level as outlet portion **58** advances distance *d*.

Alternatively, rather than adjusting the speed or the energy level, outlet portion **58** may reciprocate a multiple of times either at increasing depths or at decreasing depths. For example, referencing FIG. **12****,** if the desired depth of the hole to be formed is distance *d* (that is, the distance end surface **60** advances beyond the distal end of handpiece **54**), then tissue drill **50** may be configured to advance outlet portion **58** a distance *d* on a first stroke and then to advance outlet portion **58** a distance which incrementally decreases for each subsequent stroke for a predetermined number of strokes. Accordingly, even though the speed at which outlet portion **58** advances and the level at which laser energy L is emitted, hole **142** may be formed with a relatively large diameter at the endocardium **136** and tapered toward the epicardium **134** because tissue toward the endocardium **134** is subject to repeated laser energy with the multiple strokes of outlet portion **58.** Therefore, a greater portion of this tissue is ablated because of the increased level of energy received per unit time. Alternatively, rather than decreasing the distance of the stroke, the distance of each multiple stroke may be incrementally increased to form hole **140** of FIG. **11****.** In addition, if it is desired to form a hole with a relatively large-diameter inner chamber, then tissue drill **50** may pause outlet portion **58** at a predetermined distance for a predetermined amount of time to concentrate laser energy at one location to ablate a relatively large portion of tissue at that location.

Those skilled in the art will understand that the embodiments of the present invention described above exemplify the present invention and do not limit the scope of the invention to these specifically illustrated and described embodiments. The scope of the invention is determined by the terms of the appended claims and their legal equivalents, rather than by the described examples. In addition, the exemplary embodiments provide a foundation from which numerous alternatives and modifications may be made, which alternatives and modifications are also within the scope of the present invention as defined in the appended claims.

## Claims

1. A tissue drill (50) for reduced-trauma tissue ablation, said tissue drill (50) comprising:
an optical fiber (56) having a laser inlet end (86) and a laser outlet end (62);
a laser (52) optically coupled to said laser inlet end (86); and
a handpiece (54) comprising a body portion (64) having a distal end (68) and further comprising a coupling portion (66) for receiving said optical fiber (56) in axially movable relationship with respect to said body portion (64);
**characterized by**
the handpiece (54) being adapted to receive said optical fiber (56) in controlled, reciprocating relationship thereto, wherein said coupling portion (66) includes a drive (112) for moving the laser outlet end (62) of the optical fiber (56) to a position beyond said distal end (68), said drive (112) being configured to axially reciprocate the optical fiber (56) during ablation of said tissue by laser energy emitted from said laser outlet end (62).

2. A tissue drill (50) as claimed in claim 1 wherein
said laser outlet end (63) of said optical fiber (56) reciprocates from said distal end (68).

3. A tissue drill (50) as claimed in claim 2 wherein said laser outlet end (62) reciprocates from said distal end (68) between a plurality of positions including at least an advanced position and a retracted position.

4. A tissue drill (50) as claimed in claim 3 wherein said positions are programmable.

5. A tissue drill (50) as claimed in claim 3 wherein said handpiece (54) is adapted to receive said optical fiber (56) in controlled, rotatable relationship thereto.

6. A tissue drill (50) as claimed in claim 5 wherein said laser outlet end (62) of said optical fiber (56) is rotatable about an axis of rotation while reciprocating between said positions.

7. A tissue drill (50) as claimed in claim 6 wherein said laser outlet end (62) is eccentric with respect to said axis of rotation of said optical fiber (56).

8. A tissue drill (50) as claimed in claim 3 wherein said positions include a position in which said laser outlet end (62) is proximal to said distal end (68).

9. A tissue drill (50) as claimed in claim 1 wherein said tissue drill (50) is adapted to ablate heart tissue.

10. A tissue drill (50) as claimed in claim 9 wherein said heart tissue is myocardium.

11. A tissue drill (50) as claimed in claim 1 wherein said drive (112) moves the laser outlet end (62) of the optical fiber (56) to a position substantially at or near said distal end (68).

12. A tissue drill (50) as claimed in claim 1 wherein said drive (112) reciprocates the laser outlet end (62) of the optical fiber (56) between a plurality of positions beyond said distal end (68).

13. A tissue drill (50) as claimed in claim 1 wherein said coupling portion (66) is programmable for varying said position.

14. A tissue drill (50) as claimed in claim 1 wherein said coupling portion (66) is adapted to receive the optical fiber (56) in controlled, rotatable relationship.

15. A tissue drill (50) as claimed in claim 14 wherein said coupling portion (66) is adapted to rotate the laser outlet end (62) while moving the laser outlet end (62) to said advanced position.

16. A tissue drill as claimed in claim 15 wherein said drive (112) moves the laser outlet end (62) of the optical fiber (56) to a retracted position substantially at or near said distal end (68);
said coupling portion (66) being adapted to rotate the laser outlet end (62) while moving the laser outlet end (62) from said advanced position to said retracted position.

17. A tissue drill (50) as claimed in claim 16 wherein said drive (112) is adapted for reciprocating the laser outlet end (62) of the optical fiber (56) between said advanced position and said retracted position.

18. A tissue drill (50) as claimed in claim 17 wherein said coupling portion (66) is programmable for varying said advanced position.

19. A tissue drill (50) as claimed in claim 1 wherein said body portion (64) is a catheter.

20. A tissue drill (50) as claimed in claim 1 wherein said optical fiber (56) comprises:
an elongate portion (80) having a core (82) with said inlet (86) receiving laser energy, said core having an axis;
an outlet portion (58) having a core (82) and including said laser outlet (62) end for emitting laser energy, said core (82) of said outlet portion (58) having an axis eccentric to the axis of said core (82) of said elongate portion (80).

21. A tissue drill (50) as claimed in claim 20 wherein said outlet portion (58) has a diameter ranging from about 0.3 millimeter (mm) to about 1 mm.

22. A tissue drill (50) as claimed in claim 20 wherein said outlet portion (58) has a length of approximately 50 mm.

23. A tissue drill (50) as claimed in claim 20 wherein said elongate portion (80) has a length of approximately 2 meters.

24. A tissue drill (50) as claimed in claim 20 wherein said outlet portion (58) includes an auxiliary cladding (88) with an axis substantially concentric with the axis of said core (82) of said elongate portion (80).

25. A tissue drill (50) as claimed in claim 20 wherein said outlet portion (58) has an end surface (60) from which said laser outlet end (62) emits laser energy;
said laser outlet end (62) having a diameter of at least approximately one half of a diameter of said end surface (60).

26. A tissue drill (50) as claimed in claim 25 wherein the diameter of said end surface (60) is approximately 1 mm and the diameter of said outlet portion (58) is approximately 0.6 mm.

27. A tissue drill as claimed in claim 1 wherein the optical fiber (56) is configured to reciprocate a multiple number of times either at increasing or decreasing depths.

## Patentansprüche

1. Gewebebohrer (50) zur Gewebeablation mit verringertem Trauma, wobei der Gewebebohrer (50) umfasst:
eine optische Faser (56) mit einem Lasereinlassende (86) und einem Laserauslassende (62);
einen Laser (52), der mit dem Lasereinlassende (86) optisch gekoppelt ist; und
ein Handstück (54), das einen Körperabschnitt (64) mit einem distalen Ende (68) umfasst und ferner einen Kopplungsabschnitt (66) zum Aufnehmen der optischen Faser (56) in axial bewegbarer Beziehung in Bezug auf den Körperabschnitt (64) umfasst;
**dadurch gekennzeichnet, dass**
das Handstück (54) angepasst ist, um die optische Faser (56) in gesteuerter, sich hin- und herbewegender Beziehung aufzunehmen, wobei der Kopplungsabschnitt (66) einen Antrieb (112) aufweist, um das Laserauslassende (62) der optischen Faser zu einer Position über das distale Ende (68) hinaus zu bewegen, wobei der Antrieb (112) konfiguriert ist, um die optische Faser (56) während der Ablation des Gewebes durch von dem Laserauslassende (62) emittierter Laserenergie axial hin- und herzubewegen.

2. Gewebebohrer (50) gemäß Anspruch 1, bei dem sich das Laserauslassende (62) der optischen Faser (56) von dem distalen Ende (68) hin- und herbewegt.

3. Gewebebohrer (50) gemäß Anspruch 2, bei dem sich das Laserauslassende (62) von dem distalen Ende (68) zwischen einer Mehrzahl von Positionen einschließlich einer vorgerückten Position und einer eingezogenen Position hin- und herbewegt.

4. Gewebebohrer (50) gemäß Anspruch 3, bei dem die Positionen programmierbar sind.

5. Gewebebohrer (50) gemäß Anspruch 3, bei dem das Handstück (54) angepasst ist, um die optische Faser (56) in gesteuerter, drehbarer Beziehung aufzunehmen.

6. Gewebebohrer (50) gemäß Anspruch 5, bei dem das Laserauslassende (62) der optischen Faser (56) um eine Drehachse drehbar ist, während es sich zwischen den Positionen hin- und herbewegt.

7. Gewebebohrer (50) gemäß Anspruch 6, bei dem das Laserauslassende (62) mit Bezug auf die Drehachse der optischen Faser (56) exzentrisch ist.

8. Gewebebohrer (50) gemäß Anspruch 3, bei dem die Positionen eine Position aufweisen, bei der das Laserauslassende (62) nahe dem distalen Ende (68) ist.

9. Gewebebohrer (50) gemäß Anspruch 1, bei dem der Gewebebohrer (50) angepasst ist, um Herzgewebe zu abladieren.

10. Gewebebohrer (50) gemäß Anspruch 9, bei dem das Herzgewebe Myokardium ist.

11. Gewebebohrer (50) gemäß Anspruch 1, bei dem der Antrieb (112) das Laserauslassende (62) der optischen Faser (56) zu einer Position bewegt, die im Wesentlichen an oder nahe dem distalen Ende (68) ist.

12. Gewebebohrer (50) gemäß Anspruch 1, bei dem der Antrieb (112) das Laserauslassende (62) der optischen Faser (56) zwischen einer Mehrzahl von Positionen über das distale Ende (68) hinaus hin- und herbewegt.

13. Gewebebohrer (50) gemäß Anspruch 1, bei dem der Kopplungsabschnitt (66) zum Verändern der Position programmierbar ist.

14. Gewebebohrer (50) gemäß Anspruch 1, bei dem der Kopplungsabschnitt (66) angepasst ist, um die optische Faser (56) in gesteuerter, drehbarer Beziehung aufzunehmen.

15. Gewebebohrer (50) gemäß Anspruch 14, bei dem der Kopplungsabschnitt (66) angepasst ist, um das Laserauslassende (62) zu drehen, während das Laserauslassende (62) zu der vorgerückten Position bewegt wird.

16. Gewebebohrer (50) gemäß Anspruch 15, bei dem der Antrieb (112) das Laserauslassende (62) der optischen Faser (56) zu einer eingezogenen Position bewegt, die im Wesentlichen an oder nahe dem distalen Ende (68) ist; und
der Kopplungsabschnitt (66) angepasst ist, um das Laserauslassende (62) zu drehen, während das Laserauslassende (62) von der vorgerückten Position zu der eingezogenen Position bewegt wird.

17. Gewebebohrer (50) gemäß Anspruch 16, bei dem der Antrieb (112) zum Hin- und Herbewegen des Laserauslassendes (62) der optischen Faser (56) zwischen der vorgerückten Position und der eingezogenen Position angepasst ist.

18. Gewebebohrer (50) gemäß Anspruch 17, bei dem der Kopplungsabschnitt (66) zum Verändern der vorgerückten Position programmierbar ist.

19. Gewebebohrer (50) gemäß Anspruch 1, bei dem der Körperabschnitt (64) ein Katheter ist.

20. Gewebebohrer (50) gemäß Anspruch 1, bei dem die optische Faser (56) umfasst:
einen länglichen Abschnitt (80), der einen Kern (82) mit dem Einlass (86) zum Aufnehmen von Laserenergie aufweist, wobei der Kern eine Achse aufweist; und
einen Auslassabschnitt (58), der einen Kern (82) aufweist und das Laserauslassende (62) zum Emittieren von Laserenergie umfasst, wobei der Kern (82) des Auslassabschnitts (58) eine Achse aufweist, die zu der Achse des Kerns (82) des länglichen Abschnitts (80) exzentrisch ist.

21. Gewebebohrer (50) gemäß Anspruch 20, bei dem der Auslassabschnitt (58) einen Durchmesser aufweist, der von etwa 0,3 mm bis etwa 1 mm reicht.

22. Gewebebohrer (50) gemäß Anspruch 20, bei dem der Auslassabschnitt (58) eine Länge von ungefähr 50 mm aufweist.

23. Gewebebohrer (50) gemäß Anspruch 20, bei dem der längliche Abschnitt (80) eine Länge von ungefähr 2 m aufweist.

24. Gewebebohrer (50) gemäß Anspruch 20, bei dem der Auslassabschnitt (58) einen Hilfsmantel (88) mit einer Achse im Wesentlichen konzentrisch mit der Achse des Kerns (82) des länglichen Abschnitts (80) aufweist.

25. Gewebebohrer (50) gemäß Anspruch 20, bei dem der Auslassabschnitt (58) eine Endoberfläche (60) aufweist, von der das Laserauslassende (62) Laserenergie emittiert; und
das Laserauslassende (62) einen Durchmesser von mindestens ungefähr der Hälfte des Durchmessers der Endoberfläche (60) aufweist.

26. Gewebebohrer (50) gemäß Anspruch 25, bei dem der Durchmesser der Endoberfläche (60) ungefähr 1 mm und der Durchmesser des Auslassabschnitts (58) ungefähr 0,6 mm ist.

27. Gewebebohrer (50) gemäß Anspruch 1, bei dem die optische Faser (56) konfiguriert ist, um sich mehrfach mit zunehmender oder abnehmender Tiefe hin- und herzubewegen.

## Revendications

1. Foret pour tissu (50) destiné à l'ablation d'un tissu avec traumatisme réduit, ledit foret pour tissu (50) comprenant :
une fibre optique (56) comportant une extrémité d'entrée de laser (86) et une extrémité de sortie de laser (62) ;
un laser (52) couplé optiquement à ladite extrémité d'entrée de laser (86) ; et
une pièce à main (54) comprenant une partie formant corps (64) ayant une extrémité distale (68) et comprenant en outre une partie de couplage (66) destinée à recevoir ladite fibre optique (56) dans une relation de mobilité axiale par rapport à ladite partie formant corps (64) ;
**caractérisé en ce que**
la pièce à main (54) est apte à recevoir ladite fibre optique (56) dans une relation commandée de va-et-vient par rapport à celle-ci, ladite partie de couplage (66) comprenant une commande (112) destinée à déplacer l'extrémité de sortie de laser (62) de la fibre optique (56) vers une position située au-delà de ladite extrémité distale (68), ladite commande (112) étant configurée pour animer la fibre optique (56) d'un mouvement de va-et-vient axial pendant l'ablation dudit tissu par une énergie laser émise par ladite extrémité de sortie de laser (62).

2. Foret pour tissu (50) selon la revendication 1, dans lequel ladite extrémité de sortie de laser (62) de ladite fibre optique (56) est animée d'un mouvement de va-et-vient à partir de ladite extrémité distale (68).

3. Foret pour tissu (50) selon la revendication 2, dans lequel ladite extrémité de sortie de laser (62) est animée d'un mouvement de va-et-vient à partir de ladite extrémité distale (68) entre une pluralité de positions comprenant au moins une position avancée et une position rentrée.

4. Foret pour tissu (50) selon la revendication 3, dans lequel lesdites positions peuvent être programmées.

5. Foret pour tissu (50) selon la revendication 3, dans lequel ladite pièce à main (54) est apte à recevoir ladite fibre optique (56) dans une relation commandée et rotative par rapport à celle-ci.

6. Foret pour tissu (50) selon la revendication 5, dans lequel ladite extrémité de sortie de laser (62) de ladite fibre optique (56) peut tourner autour d'un axe de rotation tout en étant animée d'un mouvement de va-et-vient entre lesdites positions.

7. Foret pour tissu (50) selon la revendication 6, dans lequel ladite extrémité de sortie de laser (62) est excentrée par rapport audit axe de rotation de ladite fibre optique (56).

8. Foret pour tissu (50) selon la revendication 3, dans lequel lesdites positions comprennent une position dans laquelle ladite extrémité de sortie de laser (62) est à proximité de ladite extrémité distale (68).

9. Foret pour tissu (50) selon la revendication 1, dans lequel ledit foret pour tissu (50) est apte à l'ablation d'un tissu cardiaque.

10. Foret pour tissu (50) selon la revendication 9, dans lequel ledit tissu cardiaque est le myocarde.

11. Foret pour tissu (50) selon la revendication 1, dans lequel ladite commande (112) déplace l'extrémité de sortie de laser (62) de la fibre optique (56) vers une position située sensiblement au niveau ou à proximité de ladite extrémité distale (68).

12. Foret pour tissu (50) selon la revendication 1, dans lequel ladite commande (112) anime l'extrémité de sortie de laser (62) de la fibre optique (56) d'un mouvement de va-et-vient entre une pluralité de positions situées au-delà de ladite extrémité distale (68).

13. Foret pour tissu (50) selon la revendication 1, dans lequel ladite partie de couplage (66) peut être programmée pour faire varier ladite position.

14. Foret pour tissu (50) selon la revendication 1, dans lequel ladite partie de couplage (66) est apte à recevoir ladite fibre optique (56) dans une relation commandée et rotative.

15. Foret pour tissu (50) selon la revendication 14, dans lequel ladite partie de couplage (66) est apte à faire tourner l'extrémité de sortie de laser (62) tout en déplaçant l'extrémité de sortie de laser (62) vers ladite position avancée.

16. Foret pour tissu selon la revendication 15, dans lequel ladite commande (112) déplace l'extrémité de sortie de laser (62) de la fibre optique (56) vers une position rentrée, située sensiblement au niveau ou à proximité de ladite extrémité distale (68),
ladite partie de couplage (66) étant apte à faire tourner l'extrémité de sortie de laser (62) tout en déplaçant l'extrémité de sortie de laser (62) de ladite position avancée vers ladite position rentrée.

17. Foret pour tissu (50) selon la revendication 16, dans lequel ladite commande (112) est apte à animer l'extrémité de sortie de laser (62) de la fibre optique (56) d'un mouvement de va-et-vient entre ladite position avancée et ladite position rentrée.

18. Foret pour tissu (50) selon la revendication 17, dans lequel ladite partie de couplage (66) peut être programmée pour faire varier ladite position avancée.

19. Foret pour tissu (50) selon la revendication 1, dans lequel ladite partie formant corps (64) est un cathéter.

20. Foret pour tissu (50) selon la revendication 1,
dans lequel ladite fibre optique (56) comprend :
une partie allongée (80) ayant une âme (82) avec ladite entrée (86) destinée à recevoir une énergie laser,
ladite âme ayant un axe ;
une partie de sortie (58) ayant une âme (82) et comprenant ladite extrémité de sortie de laser (62) destinée à émettre une énergie laser, ladite âme (82) de ladite partie de sortie (58) ayant un axe excentré par rapport à l'axe de ladite âme (82) de ladite partie allongée (80).

21. Foret pour tissu (50) selon la revendication 20, dans lequel ladite partie de sortie (58) a un diamètre variant d'environ 0,3 millimètres (mm) à environ 1 mm.

22. Foret pour tissu (50) selon la revendication 20, dans lequel ladite partie de sortie (58) a une longueur d'environ 50 mm.

23. Foret pour tissu (50) selon la revendication 20, dans lequel ladite partie allongée (80) a une longueur d'environ 2 mètres.

24. Foret pour tissu (50) selon la revendication 20, dans lequel ladite partie de sortie (58) comprend une gaine auxiliaire (88) avec un axe sensiblement concentrique avec l'axe de ladite âme (82) de ladite partie allongée (80).

25. Foret pour tissu (50) selon la revendication 20, dans lequel ladite partie de sortie (58) comporte une surface d'extrémité (60) à partir de laquelle ladite extrémité de sortie de laser (62) émet une énergie laser ;
ladite extrémité de sortie de laser (62) ayant un diamètre d'au moins environ la moitié du diamètre de ladite surface d'extrémité (60).

26. Foret pour tissu (50) selon la revendication 25, dans lequel le diamètre de ladite surface d'extrémité (60) mesure environ 1 mm et le diamètre de ladite partie de sortie (58) mesure environ 0,6 mm.

27. Foret pour tissu selon la revendication 1, dans lequel la fibre optique (56) est configurée pour effectuer un mouvement de va-et-vient à de multiples reprises à des profondeurs croissantes ou décroissantes.
